# EUROPEAN PATENT APPLICATION

(11) **EP 4 643 635 A1**
(43) Date of publication of application: **05.11.2025**
(21) Application number: 24174225.3
(22) Date of filing: 03.05.2024
(51) Int. Cl.: A01G 25/16, G01N 33/24

(54) **METHOD OF INDICATING OVER-IRRIGATION OF SOIL, BURIABLE SOIL MONITORING DEVICE AND CORRESPONDING METHODS OF CALIBRATION**

(71) Applicant: Soil Scout Oy, 01610 Vantaa (FI)
(72) Inventor: Sirkiä, Jussi Aleksi, 21500 Piikkiö (FI); Tiusanen, Markku Johannes, 28450 Vanha-Ulvila (FI)
(74) Representative: Väisänen, Olli Jaakko

(57) **Abstract**

In order to indicate over-irrigation of soil, a method is proposed in which:
a) at least one buriable soil monitoring device (1), comprising a soil moisture monitoring part, a soil gaseous O₂ concentration monitoring part and a radio transmitter part to transmit monitoring data, is used underground to measure soil moisture within a soil moisture measurement range of the soil moisture monitoring part and also soil gaseous O₂ concentration within the same measurement range, and to transmit the monitoring data to above soil;
b) receiving and analysing the monitoring data, and based on the monitoring data,
b1) a mathematical relationship between the soil moisture level and gaseous O₂ concentration is determined and
b2) the duration of gaseous O₂ level below a limit value is determined, and

c) over-irrigation is indicated by observing which soil moisture content leads to a prolonged reduction of gaseous O₂ concentration in soil.

Independent patent claims for further methods and buriable soil monitoring devices.

## Description

### Field of the invention

The invention relates to avoiding overirrigation of soil in agriculture, sports turf maintenance and the green space landscaping industry, and to reducing hypoxia in plants.

Further, certain aspects of the invention relate to buriable soil monitoring devices, and also to methods of calibrating a buriable soil monitoring device or a group of buriable soil monitoring devices.

### Technical background

Plants simultaneously consume and produce O₂ via respiration and photosynthesis, respectively. Different crop or cropland types have dissimilar O₂ consumption rates. The ideal plant population density in agricultural practice depends on solar radiation, water and nutrient availability but also by the ratio between potential atmospheric O₂ recharge rates and its consumption by roots. [1]

It is known that irrigation respirates soil but it may also cause O₂ shortage in soil. Hypoxia (partial lack of O₂) or anoxia (complete lack of O₂) are long known to have harmful effects on plant health. [1]

For given irrigation (or precipitation) regimes and respiration rates, water contents may determine the extent and duration of hypoxia or anoxia (Ilan Ben-Noah and Shnulik P. Friedman in [1]). These are rather difficult to measure. Different soil types let water pass through at a different speed and may absorb water differently.

Soil atmosphere oxygen monitoring is normally performed using a rod-like sensor probe which is stuck in the soil and then pushed deeper to a suitable depth. The measurement results from the sensor are collected by sensor electronics which are typically located above ground and connected via wiring or cables in the rod, or a separate cable. The sensor electronics typically may include a radio communication system that transmits the sensor results using an over the air radio protocol.

In such soil monitors, the rod or the cable which connects the sensor to the sensor electronics usually extends from below soil to above soil and may act as a passage for water and atmospheric gases, thus possibly impacting the measurement results. The energy supply for the sensor electronics is typically located above earth. Typically, a rechargeable battery may be used, the recharging of which may be carried out with electricity from some sort of solar panel installation which may be integrated to the sensor electronics.

### Objective of the invention

A first objective of the invention is to help avoid the adverse effects of over-irrigation of soil in cultivation of soil and in the green field industry. This objective can be met with the method according to independent claim 1. There is a widespread understanding that over-irrigation has numerous adverse effects on plant and soil health, wastes water resources and irrigation energy, and potentially pollutes groundwaters. Just how much water is "over irrigation" is, however, not clear without measuring soil conditions. Even if soil sensors are being used to ensure that irrigation rates are kept to a dosage where no significant amounts of water are lost to deep soil layers, even those irrigation rates may already cause serious hypoxia on anoxia conditions in the root zone, but with prior methods that has been impossible to measure and detect in practical farming or in the greenkeeping industry.

A second objective of the invention is to reduce hypoxia in plants. This objective can be met with the method according to independent claim 3. There are numerous actions that a farmer or greenkeeper can take to improve the gas exchange capability of a soil, but there has been no method to accurately observe the need to carry out such actions, nor to then observe the success of such actions. The lack of data to support these decisions leads to inaccurate assessment, unnecessary actions, or in the case of not carrying out needed actions, to a lowered plant and soil health.

A third objective of the invention is to improve the reliability of measurement results of a buriable soil monitoring device. This objective can be achieved with the device according to claim 6. Devices with cable connection for data transfer or above-ground elements cannot be placed into important places of interest when there is significant activity such as tillage or mowing, and in cases where above-ground elements can be accepted, regular field operations are not conducted over the device, resulting in a non-representative location for measurements.

A fourth objective of the invention is to increase reliability of detection or reduction of hypoxia in plants. This objective can be achieved with the device according to claim 11. Devices with above-ground elements always have a rod or a cable traveling from the sensor to the above-ground atmosphere creating a passage for both surface water and gases to travel in an abnormal way, resulting in non-representative or even completely inaccurate measurements. This is most true in soils with high clay content, as those soils tend to shrink and swell depending on soil moisture content, causing a cable passage to turn into a water and gas passage.

A fifth objective of the invention is to improve long-term accuracy of buriable soil monitoring devices. This objective can be achieved with the methods according to claim 18 and the parallel independent claim 19. Once a wireless sensor is buried underground, it is laborious to bring the sensors out from the ground and a sequence of removing them from the ground and re-installing causes a disruption in the data series. A method to use only one or a subset of sensors to indicate sensor deterioration to create a calibration coefficient to all sensors staying in a buried state the data series for the whole measurement field gets disrupted and there is a significant risk of getting an undesired step change in the readings, hampering long-term analysis of the obtained soil monitoring data.

The dependent claims describe advantageous aspects of the buriable soil monitoring device and of the methods.

### Advantages of the invention

In the method of indicating over-irrigation limit of soil,
a) at least one buriable soil monitoring device, preferably according to the fourth aspect of the invention, comprising a soil moisture monitoring part and a soil gaseous O₂ concentration monitoring part and a radio transmitter part to transmit monitoring data, is used underground to measure soil moisture within a soil moisture measurement range of the soil moisture monitoring part and also soil gaseous O₂ concentration within the same measurement range, and to transmit monitoring data to above soil;
b) receiving and analysing the monitoring data, and based on the monitoring data,
   b1) a mathematical relationship between the soil moisture level and gaseous O₂ concentration is determined and
   b2) the duration of O₂ level below a limit value is determined, and
c) over-irrigation is indicated by observing which soil moisture content leads to a prolonged reduction of gaseous O₂ concentration in soil.

In this manner, hypoxia or anoxia due to soil gas exchange capability being hampered by water filled soil pores can be avoided in future, such as, by controlling the duration and/or amount of irrigation before soil moisture reaches a level that were later to cause hypoxia or anoxia.

Additionally, in the method it is possible that d) upon over-irrigation being indicated, a control device is configured, preferably automatically, to start or increase the power of at least one over-irrigation countermeasure, such as:
- by controlling operation of a water removal system to remove water from the soil using a subsurface drainage system including at least one valve and/or pump, such as by opening the at least one valve and/or starting the at least one pump or increasing the pump speed, and/or
- by controlling operating a subsurface air pumping system to increase air entering to a subsurface air piping and including at least one valve and/or pump, such as by opening the at least valve and/or starting the at least one pump or increasing the pump speed.

This kind of approach combines the use of performing gaseous O₂ concentration level measurements and the soil moisture measurements "on the spot" such that the gaseous O₂ concentration level measured is performed within the measurement range of the soil moisture measurement with an active control of actuators to carry out over-irrigation countermeasures. Because of the increased resolution of the measurement, the combination may be very powerful in the indication or reduction of hypoxia or anoxia in plants.

Because water passes soil to some extent, in some soil types much better than in some others, the onset of hypoxia or anoxia does not necessarily happen immediately after irrigation. Further, the previous irrigation or rainfall may have left soil pores filled or partially filled which makes prediction of soil moisture level that will cause anoxia or hypoxia extremely difficult if not impossible. With the present method and essentially because both soil moisture measurement and gaseous O₂ concentration level are measured within the measurement range of the soil moisture measurement unit, which typically is a few centimeters (such as, 4-6 cm, preferably around 5 cm), the measurement results relate essentially to the same spot in soil and not to separate points which, due to changes in the microstructure and water permeability of the soil may be drastically different.

In the method of preventing hypoxia in plants, gaseous O₂ concentration is monitored in soil by underground measurements, using a number of buriable soil monitoring devices preferably according to third or fourth aspect of the invention, and communicating the measurement results from below ground to above ground, receiving and analysing the measurement results, and upon determining from the measurement results that the duration of gaseous O2 level falls below a limit value, optionally for a prolonged duration, taking up a measure to increase soil oxygen exchange capability, such as loosening the soil. The analysis of the measurement results to identify hypoxia and the severity of it can be carried out manually, but it can also be implemented by means of an algorithm which can run on the sensor device itself, or alternatively or in addition, on the unit receiving the radio signals from below ground, and/or on a device receiving the results over a wired or wireless data connection, such as a mobile (e.g. cellular network) connection or the internet.

The limit value may be a known threshold of oxygen damage i.e. hypoxia to plants, preferably between 6 - 14%, more preferably 8 - 12%, most preferably around 10%.

The prolonged deficit of gaseous O₂ may be determined when gaseous O₂ concentration in soil is below the threshold limit value of hypoxia to plants or anoxia to plants at least for a duration that adversely impacts root tip survival, such as between 2 - 8 hours, preferably between 3 - 6 hours, most preferably between 4 - 5 hours.

With the method, a more reliable determination of over-irrigation can be obtained. This is partly due to the fact that both soil moisture and gaseous O₂ concentration in soil are measured "on the spot" as explained above (in this context thus advantageously minimizing the distance between these two kinds of measurement types and thus making the measurements better correlate with the actual situation at the measurement location), and partly due to the fact that there is no need for wiring or cables in the rod, nor a separate cable is necessary that would be an underground unit and above-ground parts which could act as passages for water or gases, false results that would result from water or gases being transported in the passages may be avoided.

A buriable soil atmosphere oxygen monitoring device according to the third aspect of the invention comprises i) an ultra-low power gaseous O2 concentration level sensor (such as a galvanic cell type gaseous O₂ sensor or optical sensor) for generating a voltage that is responsive to gaseous O₂ concentration at a measurement chamber comprising a measurement port, ii) an electronic circuitry electronically connected to the sensor, comprising the following iia) measurement electronics for reading the voltage, iib) an underground antenna, iic) an underground wireless radio transmitter connected to the underground antenna and configured to generate and send a signal representative to the voltage.

The transmittter comprises an antenna matching configured such that the transmitter is matched to the antenna when the oxygen monitoring device is buried in soil but in mismatch with the antenna when the oxygen monitoring device is unburied.

The monitoring device further comprises iii) a battery for energizing the measurement electronics and the underground wireless radio transmitter, and iv) a waterproof sealing that encompasses: a) the ultra-low power gaseous O₂ concentration level sensor, b) the electronic circuitry, and c) the battery, however d) leaving uncovered: d1) the measurement port of the sensor for allowing gases to diffuse between the measurement chamber and surroundings of the device and also d2) the underground antenna.

Reliability may be improved since with the monitoring device no wiring or cables in the rod, nor a separate cable is necessary between the underground unit and the above-ground parts such as transmitter and antenna since the monitoring device will be buried in soil. In this manner, they cannot act as passages for water and ambient gases and therefore, the measurement reliability may be improved.

Further, the waterproof sealing that does not encompass the measurement port and the underground antenna makes the monitoring device suitable for inhospitable conditions that may be present in soil. It is essential that the underground antenna is not encompassed by the waterproof sealing. In this manner, when the sensor device is buried in ground, the underground antenna connects to the surrounding soil and full sending power is obtained. Furthermore, it is essential that the waterproof sealing leaves the measurement port uncovered. In practice, we prefer molding chemically and mechanically durable plastics, such as polyurethane, around the monitoring device. The molding form preferably made of silicone is constructed in such a way that it prevents the antenna from getting polyurethane on it.

The type of the ultra-low power gaseous O₂ concentration level sensor that can be used in this manner is preferably a galvanic cell type gaseous O₂ sensor, such as type KE25 of manufacturer Figaro Engineering Inc. This kind of sensor may be particularly advantageous when used in a buriable soil atmosphere oxygen monitoring device since using it to collect measurement data does not consume any energy; the only energy expenditure is when the measurement data (voltage) is read from the sensor by the electronic circuitry and transmitted by the transmitter.

The sensor may comprise a heating resistance but the electronic circuitry is in this situation preferably configured not to convey electrical energy through the heating resistance, such as that would happen when electrical energy from the battery were led through the heating resistance. These kinds of O₂ sensors usually contain a resistance with which the measurement chamber may be heated to compensate for temperature and also dried.

During our tests, we found that such heating is not necessary when using a gaseous O₂ level sensor buried as long as the sensor is geometrically constructed in such a way that water ingress is minimized and in case water enters the measurement chamber there is a free path for it to drain out by gravity, which restores normal operation. Because heating is not performed, the usage of electrical energy can be reduced dramatically, which may assisted the usage time of the battery allowing for a sensor to operate maintenance free for several years. In this respect, any gaseous O2 concentration level sensor comprising a heating resistor may be considered to be an ultra-low power gaseous O2 concentration level sensor if it is operated in such a manner that the heating resistor is not used to pre-heat the measurement chamber or electrodes.

Since the temperature is relevant for the gaseous O₂ concentration level, the buriable soil monitoring devices according to the third and fourth aspects of the invention preferably comprise a temperature measurement unit having at least one temperature sensor. Then the electronic circuitry is configured to read the temperature sensor and transmit it by the transmitter to above soil, preferably with the measurement results.

Therefore, the gaseous O₂ concentration level sensor is preferably geometrically constructed to prevent water ingress such that in case water enters the measurement chamber there is a free path for the water to drain out by gravity. This easens the use of ultra-low power gaseous O₂ concentration level sensor tremendously and drastically increases the quality of the measurement results and thus also measurement reliability.

If the ultra-low power gaseous O₂ level sensor has a shape defining at least one shoulder around the measurement port, and if the electronics circuitry comprises a circuit board -that preferably is a printed circuit board- that comprises an opening or hole, respective to which the sensor is coaligned such that the measurement port overlaps the opening or hole and the at least one shoulder, preferably all shoulders, is supported by or via the circuit board, the orientation of the gaseous O₂ sensor can be fixed to the orientation of the monitoring device. Preferably, the hole is at the bottom of the monitoring device in order to avoid water possibly filling the measurement chamber through the measurement port.

The waterproof sealing preferably has around the sensor a shape configured to guide water running down along the sealing gravity-assisted away from the measurement port. Guiding water further away from measurement port reduces the possibility that water would effect the gaseous O₂ concentration measurement results.

The slope of the surface of the sealing may be selected to guide water running down gravity-assisted along the sealing away from the measurement port. Guiding water further away from measurement port reduces the possibility that water would effect the gaseous O₂ concentration measurement results.

The measurement chamber may have substantially a cylindrical form having a symmetry axis, the measurement port being located at an end of the measurement chamber. The slope of the waterproof sealing may be selected to guide water running gravity-assisted along the sealing such that the water runs along the axis further away from the measurement port. Guiding water further away from measurement port reduces the possibility that water would effect the gaseous O₂ concentration measurement results adversely such that the effect would be significant.

According to a fourth aspect of the invention,
a buriable soil monitoring device -that is preferably according to the third aspect of the invention- comprises:
- a gaseous O₂ concentration level measurement part for generating a voltage that is responsive to gaseous O₂ concentration at a measurement chamber comprising a measurement port and connected to an electronic circuitry;
- a soil moisture measurement part comprising a number of measurement electrodes electronically connected to the electronic circuitry and extending through a waterproof sealing;
- an electronic circuitry electronically connected to the gaseous O₂ concentration measurement part and the soil moisture measurement part, comprising the following:
- measurement electronics for reading the voltages from the gaseous O₂ concentration measurement part and the soil moisture measurement part;
- an underground antenna;
- an underground wireless radio transmitter connected to the underground antenna and configured to generate and send a signal representative to the voltage, wherein the transmitter comprises an antenna matching configured such that the transmitter is matched to the antenna when the soil monitoring device is buried in soil but in mismatch with the antenna when the soil monitoring device is unburied; and

- a battery for energizing the measurement electronics and the underground wireless radio transmitter;
- a waterproof sealing that encompasses: i) the gaseous O₂ concentration level sensor, ii) the electronic circuitry, and iii) the battery, however leaving uncovered: a) the measurement port of the gaseous O₂ concentration level sensor for allowing gases to diffuse between the measurement chamber and surroundings of the device and also b) the underground antenna;

The electronic circuitry is configured to read a voltage from the soil moisture measurement part that represents soil moisture and to read a voltage from gaseous O₂ concentration measurement part that represents gaseous O₂ concentration level, and to generate and transmit signals representative of the soil moisture and gaseous O₂ concentration level using the underground wireless radio transmitter and the underground antenna. Furthermore, the buriable soil monitoring device has a measurement range for soil moisture and the measurement port of the gaseous O₂ concentration sensor is located within the measurement range.

Soil moisture refers to the water content of a volume of wet soil and is not to be confused with soil humidity, which would mean the relative humidity or gaseous water vapor content of the air between solid soil particles.

The soil moisture measurement part, including the electrodes, the underground antenna and the underground radio transmitter are preferably realized as described in the European patent 3 063 886 B1 of the present applicant.

With the buriable soil monitoring device according to the fourth aspect of the invention, the reliability of detection or reduction of hypoxia in plants can be increased since the measurement of gaseous O₂ concentration is measured within the measurement range of the soil moisture measurement unit. This can eliminate the distortion that would result from the measurement results relating to spatially different locations. It is known that soil structure varies from location to location so it is important to have both gaseous O₂ concentration level measurement and soil moisture measurement "from the same spot".

With the buriable monitoring device, measurement data can thus be obtained that is essentially measured "on the spot" i.e. that both soil moisture and soil atmosphere gaseous O₂ concentration are representative to the location of the monitoring device. Further, since no wiring or cables in a rod, or a separate cable is required, that would go from below the ground to above the ground, they cannot act as passages for water and ambient gasses and therefore, the measurement reliability may also be improved also with regard to soil moisture measurement.

Further, since both soil moisture and soil atmosphere gaseous O₂ concentration are measured at the location of the monitoring device, this may improve finding causality in any conclusions made based on the monitoring results by reducing distance of the positions at which the measurements for soil moisture and soil atmosphere gaseous O₂ concentration are done, since the soil moisture and soil atmosphere gaseous O₂ concentration measurement results better relate to exactly the circumstances at the location of the monitoring device. Due to natural variations in soil structure, the probability to make inaccurate conclusions increases when soil water and gas dynamics are being sensed in separate locations, and becomes worse the further away those locations are apart.

If the electronic circuitry is configured to send the signal representative to the voltage of the sensor in the same transmitting burst as the signal representative of the soil moisture, electrical energy may be saved. This may increase the time for how long the monitoring device may be used.

The measurement electrodes and antenna preferably comprise a wing-like shape extending laterally to both sides of the monitoring device to prevent the device from losing its orientation. Preferably, the antenna is laterally on one side of the monitoring device, while the electrodes are laterally on the opposite side.

The monitoring device may have an elongated form such that the measurement electrodes and the sensor do not overlap lengthwise. This is particularly useful to improve reliability of the measurements, especially if measurement electrodes and/or antenna have wing-shape extending laterally to one or both sides of the monitoring device, since wing-shape may affect passage of gasses and water in soil and thus produce a corrupted gaseous O₂ concentration measurement result.

The device may have an elongated form such that the measurement electrodes and the sensor do not overlap lengthwise. This helps to keep the measurement results reliable: the probability that the electrodes of the soil moisture measurement unit would hamper the gaseous O₂ measurement results or vice versa can be reduced. The gaseous O₂ concentration level sensor is however placed within the measurement range of the soil moisture measurement unit.

If the monitoring device comprises an attaching means for attaching a flag, such as a piece of rope, cord or band, for finding the monitoring device buried under soil, it may be easier to find the monitoring device, especially if the field contains metal objects, as the case may be in certain areas that have been theater of war, for example, and thus may contain shrapnel and remains of ammunition which would make it more tedious to relocate the monitoring devices by using a metal detector.

The attaching means, such as a hole, is preferably located at or comprised in the underground antenna. The underground antenna is a very suitable position for attaching the flag since the antenna is less compromisable for excess water or gasses than the soil moisture measurement electrodes or especially the gaseous O₂ concentration sensor.

According to a fifth aspect of the invention, in the method of calibrating a buriable soil monitoring device, after a period of using the buriable soil monitoring device, the calibration is implemented by:
- unburying the monitoring device from soil and leaving it exposed to atmosphere at or above soil surface i.e. ambient conditions;
- collecting a group of signals, comprising at least one signal, sent by the monitoring device that is sent in antenna mismatch;
- using said signal transmitted in antenna mismatch to determine a voltage representing gaseous O₂ concentration in atmosphere at or above soil surface at an altitude from sea level at the specific location i.e. ambient conditions;
- determining a correction factor using the determined voltage and a voltage determined earlier in a similar manner representing gaseous O₂ concentration in atmosphere measured with the oxygen monitoring device; and
- using the correction factor to calibrate the buriable soil atmosphere oxygen monitoring device against aging of the galvanic cell.

Alternatively, or in addition to this, in the method of calibrating a group of buriable soil monitoring devices, the above method is used for a subgroup of buriable soil monitoring devices belonging to the group, to determine a number of correction factors in the subgroup (i.e. for each monitoring device a correction factor to correct the voltage respective to gaseous O₂ concentration in atmosphere) and using the number of correction factors to calibrate the group of buriable soil atmosphere oxygen monitoring devices that remain buried.

In both methods, the altitude may have a significant impact on the ambient O₂ concentration and it is therefore preferably taken into account when calibrating. The altitude can be measured but, since the location at which the calibration is known, alternatively to measuring the altitude of the soil surface, it can be used as a parameter. With increasing altitude, for example, the correction of atmospheric oxygen can be done to a lesser value than 21%.

The methods may help to prolong the time which the monitoring devices may be used. When galvanic cell type gaseous O₂ measurement sensors age, they produce a lesser voltage in a given gaseous O₂ concentration. The manufacturer states life expectancy of sensor to be 5 years when O2=20,9% (i.e. sea-level atmosphere), and the life expectancy is based on the time when the sensor output has decreased by 30%.

In the method where the subgroup of the monitoring devices is unburied and used to prepare calibration data, the advantage is that not all monitoring devices must be unburied but rather reliable calibration can still be achieved. This saves a lot of work if the number of monitoring devices is large. The saving may be even more critical if the monitoring devices are not attached to flags that could make their finding easier.

### List of drawings

In the following, the buriable soil monitoring devices and methods are described in more detail by means of examples in the attached drawings, of which show:
- FIG 1: a buriable soil monitoring device (perspective view);
- FIG 2: the buriable soil monitoring device of FIG 1 (transparent view);
- FIG 3: the buriable soil monitoring device of FIG 1 (transparent view);
- FIG 4: bottom view of the buriable soil monitoring device of FIG 1;
- FIG 5: horizontal section V-V (cf. FIG 2) of the buriable soil monitoring device of FIG 1;
- FIG 6: a tilted section of the buriable soil monitoring device of FIG 1, illustrating the surface shape;

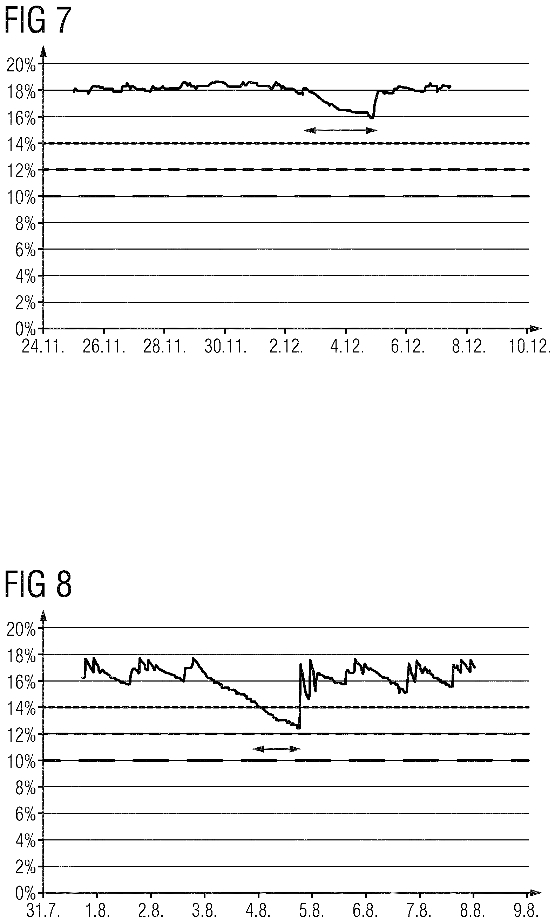

- FIG 7: example of soil oxygen measurements during an irrigation event that did not cause hypoxia;
- FIG 8: example of soil oxygen measurements during an irrigation causing mild hypoxia with O₂ level lower than 14% with a severe duration exceeding 8 hours; and

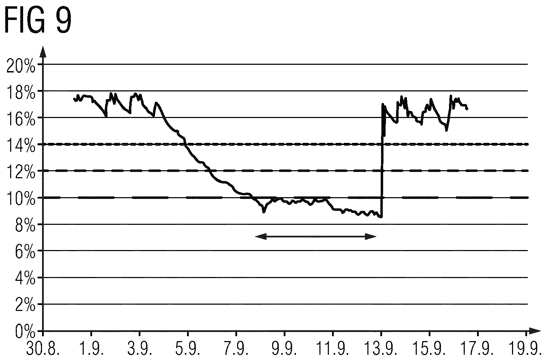

- FIG 9: example of soil oxygen measurements during an irrigation and rain resulting causing severe hypoxia lower than 10% with a severe duration exceeding several days.

Same reference numerals refer to same components in all FIG.

### Detailed description

### I Buriable soil monitoring device

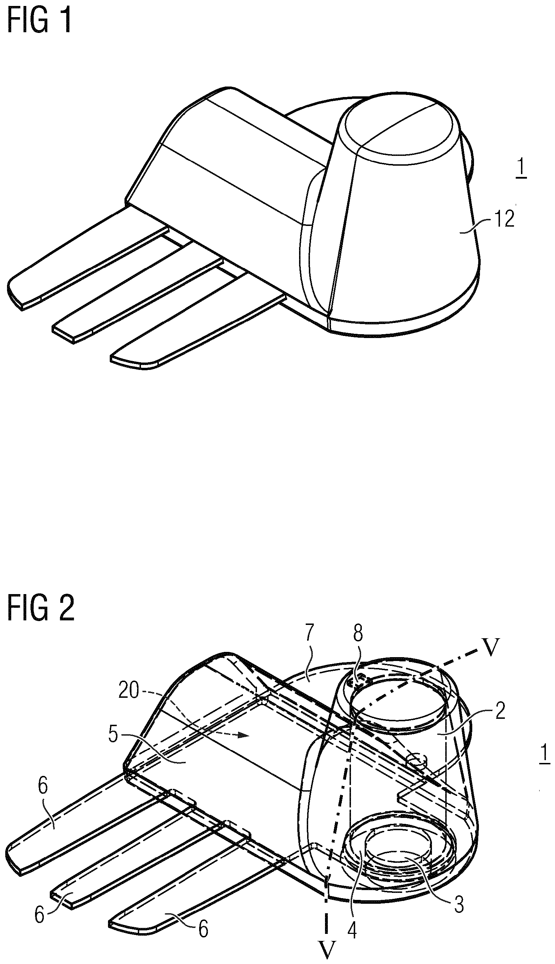

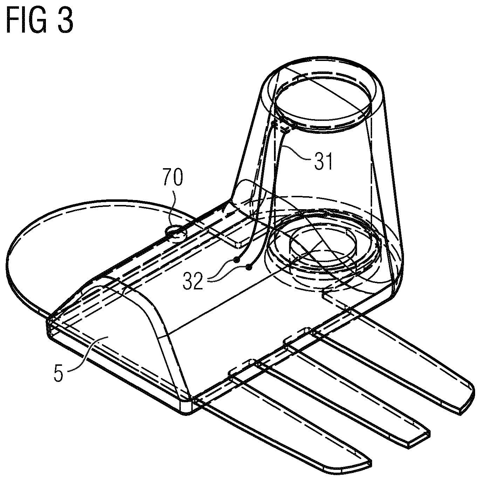

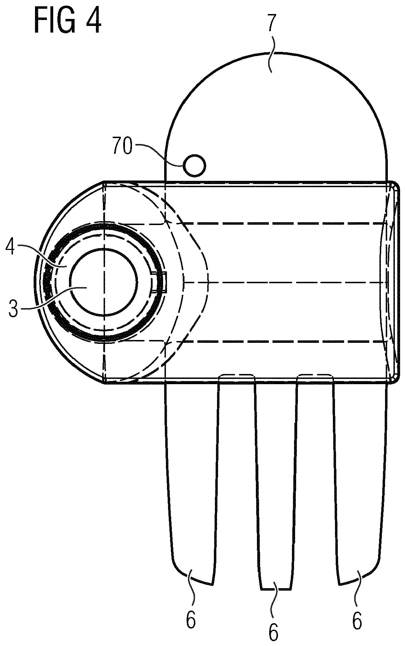

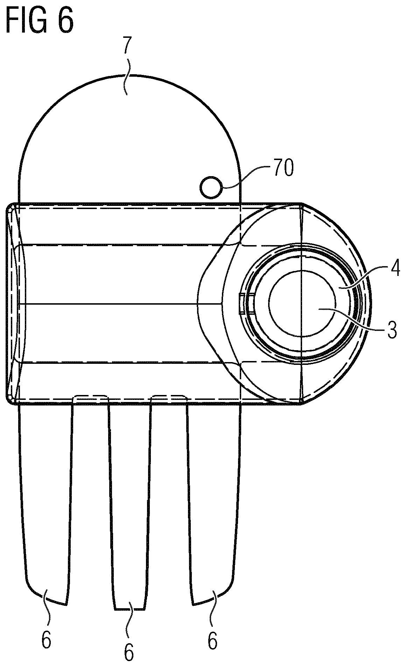

FIG 1 - 6 show a buriable soil monitoring device 1. It comprises an ultra-low power gaseous O₂ concentration level sensor 2, which in particular may be or comprise a galvanic cell type gaseous O₂ sensor. The ultra-low power gaseous O₂ concentration level sensor is for generating a voltage that is responsive to gaseous O₂ concentration at a measurement chamber comprising a measurement port 3.

The buriable soil monitoring device 1 further comprises an electronic circuitry 5 electronically connected to the sensor 2. The electronic circuitry 5 comprises the following:
- measurement electronics for reading the voltage;
- an underground antenna 7;
- an underground wireless radio transmitter 5 connected to the underground antenna 7 and configured to generate and send a signal representative to the voltage, wherein the transmitter comprises an antenna matching configured such that the transmitter is matched to the antenna when the soil monitoring device is buried in soil but in mismatch with the antenna when the soil monitoring device is unburied; and
- a battery 20 for energizing the measurement electronics and the underground wireless radio transmitter;
- a waterproof sealing 12 that encompasses:
   i) the ultra-low power gaseous O2 level sensor 2,
   ii) the electronic circuitry 5, and
   iii) the battery 20,
      however leaving uncovered:
      a) the measurement port 3 of the sensor 2 for allowing gases to diffuse between the measurement chamber and surroundings of the device 1 and also
      b) the underground antenna 7.

The ultra-low power gaseous O₂ level sensor 2 is preferably geometrically constructed to prevent water ingress such that in case water enters the measurement chamber there is a free path for the water to drain out by gravity.

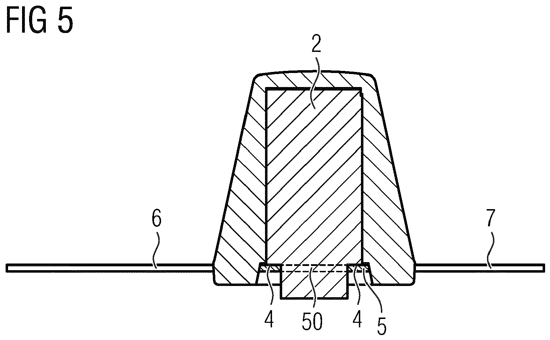

The sensor 2 may have a shape defining at least one shoulder 4 around the measurement port 3. The electronics circuitry 5 preferably comprises a circuit board -that may be a printed circuit board- that comprises an opening or hole 50 (cf. FIG 5, between shoulders 4), respective to which the sensor 2 is coaligned such that the measurement port 3 overlaps the opening or hole 50 and the at least one shoulder 4, preferably all shoulders is supported by or via the circuit board.

The waterproof sealing 12 may have around the sensor 2 a shape configured to guide water running down along the sealing gravity-assisted away from the measurement port 3.

In addition or alternatively, the slope of the surface of the sealing 12 may be selected to guide water running down gravity-assisted along the sealing 12 away from the measurement port 3.

In addition or alternatively, the measurement chamber may have substantially a cylindrical form having a symmetry axis, the measurement port 3 being located at an end of the measurement chamber. The slope of the waterproof sealing 12 may be selected to guide water running gravity-assisted along the sealing 12 such that the water runs along the axis further away from the measurement port 3.

According to a further aspect that preferably may be combined with the above explanation of the buriable soil monitoring device 1,
a buriable soil monitoring device 1 comprises:
- a gaseous O₂ concentration level measurement part 2, comprising a gaseous O₂ concentration level sensor (which is preferably a ultra-low power gaseous O₂ concentration level sensor as explained above), for generating a voltage that is responsive to gaseous O₂ concentration at a measurement chamber comprising a measurement port 3 and connected to an electronic circuitry 5;
- a soil moisture measurement part comprising a number of measurement electrodes 6 electronically connected to the electronic circuitry 5 and extending through a waterproof sealing 12;
- an electronic circuitry 5 electronically connected to the gaseous O₂ concentration level measurement part 2 and the soil moisture measurement part, comprising the following:
   - measurement electronics for reading the voltages from the gaseous O₂ concentration measurement part 2 and the soil moisture measurement part;
   - an underground antenna 7;
   - an underground wireless radio transmitter 5 connected to the underground antenna 7 and configured to generate and send a signal representative to the voltage. The transmitter comprises an antenna matching configured such that the transmitter is matched to the antenna when the soil monitoring device is buried in soil but in mismatch with the antenna when the soil monitoring device is unburied; and
- a battery 20 for energizing the measurement electronics and the underground wireless radio transmitter;
- a waterproof sealing 12 that encompasses: i) the gaseous O₂ concentration level sensor 2, ii) the electronic circuitry 5, and iii) the battery 20, however leaving uncovered: a) the measurement port 3 of the gaseous O₂ concentration level sensor 2 for allowing gases to diffuse between the measurement chamber and surroundings of the device 1 and also b) the underground antenna 7.

The electronic circuitry 5 is configured to read a voltage from the soil moisture measurement part that represents soil moisture and to read a voltage from gaseous O₂ concentration measurement part that represents gaseous O₂ concentration level, and to generate and transmit signals representative of the soil moisture and gaseous O₂ concentration level using the underground wireless radio transmitter 5 and the underground antenna 7.

The device 1 has a measurement range for soil moisture and the measurement port 3 of the gaseous O₂ concentration sensor 2 is located within the measurement range. The geometry of the moisture sensing head on the device may be of such a configuration that it creates an electric field with a given radius (such as, 50 mm) around the sensor pins. This can be considered the range of influence, and an oxygen concentration measurement performed closer than the given radius from the sensing head is obtained from the same point with the same characteristics.

The electronic circuitry 5 may be configured to send the signal representative to gaseous O₂ concentration level in the same transmitting burst as the signal representative of the soil moisture.

The measurement electrodes 6 and antenna 7 may comprise a wing-like shape extending laterally to both sides of the device 1 to prevent the device 1 from losing its orientation.

The device 1 may have an elongated form such that the measurement electrodes 6 and the sensor 2 do not overlap lengthwise.

The monitoring device 1 may comprise an attaching means 70 for attaching a flag, such as a piece of rope, cord or band, for easier finding the device 1 buried under soil. The attaching means, such as a hole 70, is preferably located at or comprised in the underground antenna 7.

### II Methods for indication

### and prevention of hypoxia in plants

According to the first aspect of the invention, in the method of indicating over-irrigation of soil,
a) at least one buriable soil monitoring device 1, preferably according the fourth aspect of the invention, comprising a soil moisture monitoring part, a soil gaseous O₂ concentration monitoring part and a radio transmitter part to transmit monitoring data, is used underground to measure soil moisture within a soil moisture measurement range of the soil moisture monitoring part and also soil gaseous O₂ concentration within the same measurement range, and to transmit the monitoring data to above soil;
b) the monitoring data is received with an electronic radio receiver device and analyzed either on the receiver device or a server computer that can communicate with the receiver device, and based on the monitoring data,
b1) a mathematical relationship between the soil moisture level and gaseous O₂ concentration is determined and
b2) the duration of gaseous O₂ level below a limit value is determined, and
c) over-irrigation is indicated by observing which soil moisture content leads to a prolonged reduction of gaseous O₂ concentration in soil.

Further, optionally:
d) upon over-irrigation being indicated, a control device is configured, preferably automatically, to start or increase the power of at least one over-irrigation countermeasure, such as:
   - by controlling operation of a water removal system to remove water from the soil using a subsurface drainage system including at least one valve and/or pump, such as by opening the at least one valve and/or starting the at least one pump or increasing the pump speed, and/or
   - by controlling operating a subsurface air pumping system to increase air entering to a subsurface air piping and including at least one valve and/or pump, such as by opening the at least valve and/or starting the at least one pump or increasing the pump speed.

According to the second aspect of the invention, in the method of preventing hypoxia in plants, gaseous O₂ concentration is monitored in soil by underground measurements, using a number of buriable soil monitoring devices (that preferably are according to the third or fourth aspects of the invention), and communicating the measurement results from below ground to above ground, receiving and analysing the measurement results, and upon determining from the measurement results that the duration of gaseous O₂ level falls below a limit value, optionally for a prolonged duration, taking up a measure to increase soil oxygen exchange capability, such as loosening the soil.

Commonly to both methods, the limit value for gaseous O₂ level may be a threshold of oxygen damage i.e. hypoxia to plants or anoxia to plants, preferably between 6 - 14%, more preferably 8 - 12%, most preferably 10%.

Commonly to both methods, the prolonged deficit of gaseous O₂ may be determined when gaseous O₂ concentration in soil is below the threshold limit value of hypoxia to plants or anoxia to plants at least for a duration that adversely impacts root tip survival, such as between 2-8 hours, preferably between 3-6 hours, most preferably between 4 - 5 hours.

Both methods proposed here utilise underground gaseous oxygen sensors to accurately measure oxygen concentrations in the root zone of plants, thereby assessing the presence and severity of hypoxia. This approach builds upon previous research findings:
Friedman & Naftaliev (2012) observed oxygen concentrations in orchard soils, revealing that at active root depths, oxygen levels typically exceeded 15%, albeit decreasing linearly with depth. This establishes a baseline understanding of oxygen distribution in the root zone. [2]

Stepniewski (1992) conducted greenhouse trials indicating that when soil oxygen levels dropped, winter rye yield at full maturity decreased significantly, even to as little as 30-50% of controls. Early onset of soil hypoxia resulted in reduced straw weight, emphasizing the detrimental impact on crop productivity. [3]

Yalin et al. (2021) identified a negative correlation between yield and the duration of oxygen levels below 10%, a threshold considered damaging to plants. This underscores the importance of monitoring and mitigating hypoxic conditions to optimize crop yield. [4]

Costello et al. (1991) demonstrated the dependency of root growth on soil oxygen levels, with root growth inhibited at concentrations below 10%. This highlights the critical role of adequate oxygen supply in supporting root development and overall plant health. [5]

Ben-Noah et al. (2018) investigated the effects of anoxia on banana root growth, finding that while immediate cessation occurred, re-aeration after specific durations allowed for partial recovery of root growth rates. Prolonged anoxic conditions resulted in irreversible damage, indicating the need for timely intervention to prevent long-term detrimental effects. [1]

As a conclusion, by integrating these findings, our proposed method offers a novel approach to assessing and managing soil oxygen levels in real-time or almost in real-time. This advancement enables precise monitoring of hypoxic conditions, facilitating informed agronomic decision-making to optimize crop productivity and mitigate yield losses associated with oxygen deficit in the root zone. Hypoxia is often caused by irrigation and/or rain, as excess water fills up the soil pore space and prevents the root zone from efficiently exchanging gas with the free atmosphere.

Until now, there has been no procedure to simultaneously measure both soil moisture and gaseous oxygen concentration in the very same point in an underground location that is managed the same way as if there was no monitoring device present. This is a key feature of our invention in two ways: 1) soil structure and texture varies within all fields and independent observations of soil moisture and oxygen concentration from separate locations, even just a little apart, would not create a consistent pair and would lead to inaccurate conclusions, and 2) measuring those soil parameters in a location with an above-ground element such as a stick, box or antenna would prevent from managing the measurement location in a representative way, such as mowing the grass or harrowing the soil over the sensor, resulting in having distorted data from a point that does not in fact represent what the soil and crop around the location is in fact experiencing.

The novel combination of an underground radio transmitter with ultra-low power oxygen sensor and soil moisture sensor can for the first time deliver long-lasting real-time soil data that allows the recognition of the severity and duration of hypoxia events and linking their assisted2424 with the observed soil moisture dynamics. As a result, the invention can for the first time show detailed data on the well known but hard-to-measure adverse effects of over irrigation and give the needed insight for agronomists to both adjust the irrigation rates and durations, as well as to take other counter measures to improve the soil's gas exchange capability, and get a reliable feedback on the effectiveness of such efforts. In addition, the soil moisture data from the very same device provides needed data to avoid under-irrigation as well, so the invention can be described as an instrument to completely manage the full spectrum of irrigation control.

According to the metioned scientific papers, it can be deducted that while 10% oxygen concentration is a severe hypoxia threshold, the concentration should in general stay above 15%. Based on these findings we have concluded that oxygen concentration below 14% is a mild hypoxia that should be avoided but causes no permanent damage to plants, below 12% is a significant hypoxia where vital functions are restricted, and below 10% is a severe hypoxia that may cause irreversible damage to plant roots.

Furthermore, according to the same papers, plant roots typically can survive a 4 hour hypoxia, while recovery after 8 hours becomes uncertain. This is the basis for our conclusion that hypoxia duration of 4 hours is a mild hypoxia, 6 hours is significant, and 8 hours is severe. Some plant species and varieties have different hypoxia tolerance and the durations need to be adjusted accordingly, which however only impacts the assisted24 parameters and has no impact on the method itself.

### III Methods for calibration

There are four important values related to oxygen sensor calibration:
1. incoming O₂ reading (o2in)
2. calibration value (CalVal) at the time (percentage 0-100%)
3. calibrated O₂ result (o2cal) (percentage 0-100%)
4. earth atmosphere oxygen content at sea level, which is a constant 20.9%. Between sea level and 3000m altitude the atmospheric oxygen content is approximated with a linear approximation according to: 20.9-0.002165 × altitude (meters).

The sensor degrades when exposed to oxygen and thus the sensor reading will decrease over time. That is why CalVal is needed. A typical scenario:
1. Sensor is fresh from the factory, in open air of 20.9% oxygen content it says 0.209 and CalVal is set to 20.9% -> o2cal=2 0.9%.
2. Sensor is buried, plants consume oxygen from the root zone, o2in drops to 0.105, o2cal=10.5%. Correct.
3. Much later, the sensor is dug up, and in air it now says 0.180 -> 02cal=18%. Error!
4. CalVal is manually set to 18.0% and 02cal is returned to 20.9%.
5. No back calculations of previous readings are performed when CalVal is changed, they remain as they were with the CalVal that was in effect at the time of readings, but when higher accuracy is needed, they can be adjusted accordingly afterwards by using a time coefficient.

Example: customer is at 2000 meters, oxygen content is 16,6%, Scout last o2cal=16.6%. Customer gives CalVal=16.6% and Alt=2000m. CalVal_out=0.166*20.9%/(0.209-0.00002165×2000)=20.9%. This makes sense as this example was based on a factory fresh sensor.

A comparison of the average hourly oxygen percentage across the calibrated sensor measurement history and another buried sensor at the site can then be used as a factor to define the needed CalVal for each sensor staying in ground. Example: the CalVal for the calibrated sensor is 16,6% and the average hourly oxygen level for the sensor history has been 18%. A buried sensor at the same site has a measurement history average of 19%, so the aging of the sensor has been 19%/18% = 1.056 times faster. The CalVal for the calibrated sensor had declined 4.3%-units from 20.9%, so the CalVal for the other sensor is 20.9%-(1.056x4.3%) = 16.34%.

In the method of calibrating a buriable soil monitoring device 1 that preferably is according to third or fourth aspect of the invention, after a period of using the soil atmosphere oxygen monitoring device 1, the calibration is implemented by:
- unburying the monitoring device 1 from soil and leaving it exposed to atmosphere at or above soil surface i.e. ambient conditions;
- collecting a group of signals, comprising at least one signal, sent by the monitoring device 1 that is sent in antenna mismatch;
- using said signal transmitted in antenna mismatch to determine a voltage representing gaseous O₂ concentration in atmosphere at or above soil surface at an altitude from sea level at the specific location i.e. ambient conditions;
- determining a correction factor using the determined voltage and a voltage determined earlier in a similar manner representing gaseous O₂ concentration in atmosphere measured with the oxygen monitoring device 1; and
- using the correction factor to calibrate the buriable soil atmosphere oxygen monitoring device 1 against aging of the galvanic cell.

In the method of calibrating a group of buriable soil monitoring devices 1 that preferably are according to third or fourth aspect of the present invention, the previous calibration method is used for a subgroup of buriable soil monitoring devices 1 belonging to the group, to determine a number of correction factors. Then in the method the number of correction factors or a value derived therefrom, such as an arithmethic mean and/or an geometrical mean, is used to calibrate the group of buriable soil monitoring devices 1 that remain buried.

The invention should not be understood to be limited only by the below claims, but the invention is to be understood to include all their legal equivalents and the combinations of the embodiments presented.

### List of reference numerals

- 1: buriable soil monitoring device
- 2: ultra-low energy gaseous O₂ concentration level sensor
- 3: measurement port
- 4: shoulder
- 5: electronics circuitry (comprising underground radio transmitter)
- 6: measurement electrode
- 7: underground antenna
- 8: electrical connector
- 12: waterproof sealing
- 20: battery
- 31: electrical wiring or cable
- 32: electrical connections
- 50: opening or hole
- 70: attaching means for attaching a flag, e.g. hole

### List of reference publications

[1] Ilan Ben-Noah, Shmulik P. Friedman, Review and Evaluation of Root Respiration and of Natural and Agricultural Processes of Soil Aeration, Vadose Zone Journal Vol 17(1), p.1-47, https://doi.org/10.2136/vzj2017.06.0119
[2] Friedman & Naftaliev (2012), A survey of the aeration status of drip-irrigated orchards, Agricultural Water Management vol 15, p. 132-147
[3] Stepniewski et al. (1992), The influence of soil oxygen availability on yield and nutrient uptake (P, K, Ca, Mg, Na) by winter rye, Plant and Soil 143 (267-274), Kluwer Academic Publishers
[4] Yalin et al. (2021), Soil oxygen and water dynamics underlying hypoxic conditions in the root-zone of avocado irrigated with treated wastewater in clay soil, Soil and Tillage Research Vol. 212, https://doi.org/10.1016/j.still.2021.105039
[5] Costello et al. (1991), Soil Aeration and Tree Health: Correlating Soil Oxygen Measurements With the Decline of Established Oaks, USDA Forest Service Gen. Tech. Rep. PSW-126

## Claims

1. A method of indicating over-irrigation of soil, **wherein:**
a) at least one buriable soil monitoring device (1), preferably according to any one of the claims 11 to 14 and optionally also with any one of the claims 15 or 16, comprising a soil moisture monitoring part, a soil gaseous O₂ concentration monitoring part and a radio transmitter part to transmit monitoring data, is used underground to measure soil moisture within a soil moisture measurement range of the soil moisture monitoring part and also soil gaseous O₂ concentration within the same measurement range, and to transmit the monitoring data to above soil;
b) receiving and analysing the monitoring data, and based on the monitoring data,
b1) a mathematical relationship between the soil moisture level and gaseous O₂ concentration is determined and
b2) the duration of gaseous O₂ level below a limit value is determined, and
c) over-irrigation is indicated by observing which soil moisture content leads to a prolonged reduction of gaseous O₂ concentration in soil.

2. The method according to claim 1, **wherein:**
d) upon over-irrigation being indicated, a control device is configured, preferably automatically, to start or increase the power of at least one over-irrigation countermeasure, such as:
- by controlling operation of a water removal system to remove water from the soil using a subsurface drainage system including at least one valve and/or pump, such as by opening the at least one valve and/or starting the at least one pump or increasing the pump speed, and/or
- by controlling operating a subsurface air pumping system to increase air entering to a subsurface air piping and including at least one valve and/or pump, such as by opening the at least valve and/or starting the at least one pump or increasing the pump speed.

3. Method of preventing hypoxia in plants, **wherein:** gaseous O₂ concentration is monitored in soil by underground measurements, using a number of buriable soil monitoring devices preferably according to any one of claims 6 to 16, and communicating the measurement results from below ground to above ground, receiving and analysing the measurement results, and upon determining from the measurement results that the duration of gaseous O₂ level falls below a limit value, optionally for a prolonged duration, taking up a measure to increase soil oxygen exchange capability, such as loosening the soil.

4. The method according to any one of the preceding claims 1 to 3, **wherein:** the limit value for gaseous O₂ level is a threshold of oxygen damage i.e. hypoxia to plants or anoxia to plants, preferably between 6 - 14%, more preferably 8 - 12%, most preferably 10%.

5. The method according to any one of the preceding claims 1 to 4, **wherein:** the prolonged deficit of gaseous O₂ is determined when gaseous O₂ concentration in soil is below the threshold limit value of hypoxia to plants or anoxia to plants at least for a duration that adversely impacts root tip survival, such as between 2-8 hours, preferably between 3-6 hours, most preferably between 4 - 5 hours.

6. A buriable soil monitoring device (1), **comprising:**
- an ultra-low power gaseous O₂ concentration level sensor (2), in particular a galvanic cell type gaseous O₂ sensor, for generating a voltage that is responsive to gaseous O₂ concentration at a measurement chamber comprising a measurement port (3);
- an electronic circuitry (5) electronically connected to the sensor (2), comprising the following:
- measurement electronics for reading the voltage;
- an underground antenna (7);
- an underground wireless radio transmitter (5) connected to the underground antenna (7) and configured to generate and send a signal representative to the voltage, wherein the transmitter comprises an antenna matching configured such that the transmitter is matched to the antenna when the soil monitoring device is buried in soil but in mismatch with the antenna when the soil monitoring device is unburied; and
- a battery (20) for energizing the measurement electronics and the underground wireless radio transmitter;
- a waterproof sealing (12) that encompasses: i) the ultra-low power gaseous O₂ level sensor (2), ii) the electronic circuitry (5), and iii) the battery (20), however leaving uncovered: a) the measurement port (3) of the sensor (2) for allowing gases to diffuse between the measurement chamber and surroundings of the device (1) and also b) the underground antenna (7).

7. The buriable soil monitoring device (1) of claim 6, **wherein:** the gaseous O₂ concentration level sensor (2) is geometrically constructed to prevent water ingress such that in case water enters the measurement chamber there is a free path for the water to drain out by gravity.

8. The buriable soil monitoring device (1) according to claim 6 or 7, **wherein:** the ultra-low power gaseous O₂ level sensor (2) has a shape defining at least one shoulder (4) around the measurement port (3), and wherein the electronics circuitry (5) comprises a circuit board -that preferably is a printed circuit board- that comprises an opening or hole (50), respective to which the sensor (2) is coaligned such that the measurement port (3) overlaps the opening or hole (50) and the at least one shoulder (4), preferably all shoulders is supported by or via the circuit board.

9. The buriable soil monitoring device (1) according to any one of the preceding claims 6 to 8, **wherein:** the waterproof sealing (12) has around the sensor (2) a shape configured to guide water running down along the sealing gravity-assisted away from the measurement port (3).

10. The buriable soil monitoring device (1) according to any one of claims 6 to 9, **wherein:** the slope of the surface of the sealing (12) is selected to guide water running down gravity-assisted along the sealing (12) away from the measurement port (3) .

11. The buriable soil monitoring device (1) according to any one of claims 6 to 10, **wherein:** the measurement chamber has substantially a cylindrical form having a symmetry axis, the measurement port (3) being located at an end of the measurement chamber, and wherein the slope of the waterproof sealing (12) is selected to guide water running gravity-assisted along the sealing (12) such that the water runs along the axis further away from the measurement port (3).

12. A buriable soil monitoring device (1) that is preferably according to any one of the preceding claims 6 to 11, **comprising:**
- a gaseous O₂ concentration level measurement part (2), comprising a gaseous O₂ concentration level sensor, for generating a voltage that is responsive to gaseous O₂ concentration at a measurement chamber comprising a measurement port (3) and connected to an electronic circuitry (5);
- a soil moisture measurement part comprising a number of measurement electrodes (6) electronically connected to the electronic circuitry (5) and extending through a waterproof sealing (12);
- an electronic circuitry (5) electronically connected to the gaseous O₂ concentration level measurement part (2) and the soil moisture measurement part, comprising the following:
- measurement electronics for reading the voltages from the gaseous O₂ concentration measurement part (2) and the soil moisture measurement part;
- an underground antenna (7);
- an underground wireless radio transmitter (5) connected to the underground antenna (7) and configured to generate and send a signal representative to the voltage, wherein the transmitter comprises an antenna matching configured such that the transmitter is matched to the antenna when the soil monitoring device is buried in soil but in mismatch with the antenna when the soil monitoring device is unburied; and
- a battery (20) for energizing the measurement electronics and the underground wireless radio transmitter;
- a waterproof sealing (12) that encompasses: i) the gaseous O₂ concentration level sensor (2), ii) the electronic circuitry (5), and iii) the battery (20), however leaving uncovered: a) the measurement port (3) of the gaseous O₂ concentration level sensor (2) for allowing gases to diffuse between the measurement chamber and surroundings of the device (1) and also b) the underground antenna (7);
and wherein: the electronic circuitry (5) is configured to read a voltage from the soil moisture measurement part that represents soil moisture and to read a voltage from gaseous O₂ concentration measurement part that represents gaseous O₂ concentration level, and to generate and transmit signals representative of the soil moisture and gaseous O₂ concentration level using the underground wireless radio transmitter (5) and the underground antenna (7) ;
and wherein: the device (1) has a measurement range for soil moisture and the measurement port (3) of the gaseous O₂ concentration sensor (2) is located within the measurement range.

13. The buriable soil monitoring device (1) according to claim 11, **wherein:** the electronic circuitry (5) is configured to send the signal representative to gaseous O₂ concentration level in the same transmitting burst as the signal representative of the soil moisture.

14. The buriable soil monitoring device (1) according to any one of the preceding claims 12 or 13, **wherein:** the measurement electrodes (6) and antenna (7) comprise a wing-like shape extending laterally to both sides of the device (1) to prevent the device (1) from losing its orientation.

15. The buriable soil monitoring device (1) according to any one of the preceding claims 12 to 14, **wherein:** the device (1) has an elongated form such that the measurement electrodes (6) and the sensor (2) do not overlap lengthwise.

16. The buriable soil monitoring device (1) according to any one of the preceding claims 6 to 15, **wherein:** the monitoring device (1) comprises an attaching means (70) for attaching a flag, such as a piece of rope, cord or band, for finding the device (1) buried under soil.

17. The buriable soil monitoring device (1) according to claim 15, **wherein:** the attaching means, such as a hole (70), is located at or comprised in the underground antenna (7).

18. The method of calibrating a buriable soil monitoring device (1) of any one of the preceding claims 6 to 17, **wherein:** after a period of using the soil atmosphere oxygen monitoring device (1), the calibration is implemented by:
- unburying the monitoring device (1) from soil and leaving it exposed to atmosphere at or above soil surface i.e. ambient conditions;
- collecting a group of signals, comprising at least one signal, sent by the monitoring device (1) that is sent in antenna mismatch;
- using said signal transmitted in antenna mismatch to determine a voltage representing gaseous O₂ concentration in atmosphere at or above soil surface at an altitude from sea level at the specific location i.e. ambient conditions;
- determining a correction factor using the determined voltage and a voltage determined earlier in a similar manner representing gaseous O₂ concentration in atmosphere measured with the oxygen monitoring device (1); and
- using the correction factor to calibrate the buriable soil atmosphere oxygen monitoring device (1) against aging of the galvanic cell.

19. A method of calibrating a group of buriable soil monitoring devices (1) of any one of the preceding claims 6 to 16, **wherein:**
the method of claim 17 is used for a subgroup of buriable soil monitoring devices (1) belonging to the group, to determine a number of correction factors; and in the method the number of correction factors or a value derived therefrom, such as an arithmethic mean and/or an geometrical mean, is used to calibrate the group of buriable soil monitoring devices (1) that remain buried.
